Europäisches Patentamt

European Patent Office

Office européen des brevets

(11) Publication number: **0 104 877**
**B1**

(12) **EUROPEAN PATENT SPECIFICATION**

(45) Date of publication of patent specification: **17.01.90**

(51) Int. Cl.⁵: **A 61 K 31/21, A 61 K 47/00**

(21) Application number: **83305558.5**

(22) Date of filing: **21.09.83**

(54) Organic nitrate drug mixtures resistant to detonation by fire.

(30) Priority: **29.09.82 US 428378**

(43) Date of publication of application:
**04.04.84 Bulletin 84/14**

(45) Publication of the grant of the patent:
**17.01.90 Bulletin 90/03**

(84) Designated Contracting States:
**AT BE CH DE FR GB IT LI LU NL SE**

(56) References cited:
**FR-A-1 098 116**
**US-A-3 096 242**
**US-A-3 905 846**

(73) Proprietor: **ICI AMERICAS INC.**
**Concord Pike & New Murphy Road**
**Wilmington Delaware 19897 (US)**

(72) Inventor: **Lynch, Matthew James**
**1200 Graylyn Road**
**Wilmington Delaware 19879 (US)**

(74) Representative: **Slatcher, Reginald Peter et al**
**Imperial Chemical Industries PLC Legal**
**Department: Patents P.O. Box 6 Bessemer Road**
**Welwyn Garden City AL7 1HD (GB)**

Courier Press, Leamington Spa, England.

**Description**

This invention is directed to drug mixtures containing high concentrations of organic nitrate compounds such as nitroglycerin dispersed in an inert excipient, and a minor amount of an alkaline solid which acts as a detonation desensitizer when the mixture is burned under oxygen-deficient conditions. The nitrate mixtures, which include isosorbide dinitrate, isosorbide 5-mononitrate, isosorbide 2-mononitrate, mannitol hexanitrate and pentaerythritol tetranitrate (PETN), are used in more dilute concentrations in preparing tablets which are used as vasodilating drugs, especially in the treatment of angina. Alkaline solid desensitizers may be selected from alkali and alkaline earth metal carbonates, bicarbonates, hydroxides and hydrous oxides, and include such materials as ammonium carbonate.

Detonatable organic nitrates such as nitroglycerin, isosorbide dinitrate, isosorbide 5-mononitrate, isosorbide 2-mononitrate, mannitol hexanitrate and PETN have been dispersed in concentrations ranging from 5—50% by weight in organic excipients such as lactose, starch, flour, mannitol, sorbitol, sugar and polyvinylpyrrolidone, and inorganic materials such as bentonite, attapulgite and silica. In particular it is known from US Specification No. 3096242 that the vasodilator substance PETN may be desensitized from percussion by formulating it as a composition containing one or more substances selected from lactose, mannitol and a finely divided highly porous material such as an activated clay, for example fuller's earth and attapulgus clay containing calcium, magnesium or aluminum silicates. Such materials are thereafter diluted further when tablets are made for medicinal use. Blends of concentrated organic nitrates are generally considered stable and do not detonate even when submitted to high energy shock waves such as that supplied by a number 8 blasting cap. Furthermore, concentrated organic nitrate/excipient blends have been considered safe and have been burned under oxygen rich conditions without incident. Waste materials containing the nitrate have for some time been disposed of by burning without incident. Recently, however, it was discovered that a commercially-available drug mixture containing 10% nitroglycerine and 90% lactose could be detonated when burned in oxygen deficient, scorching and smoldering fires.

It is the object of this invention, therefore, to provide an organic drug mixture resistant to detonation in smoldering fires which comprises:—

(a) 5—50% by weight of an organic nitrate selected from nitroglycerin, isosorbide dinitrate, isosorbide 5-mononitrate, isosorbide 2-mononitrate, mannitol hexanitrate and pentaerythritol tetranitrate,

(b) an inert nonalkaline excipient, and

(c) an effective amount of a solid alkaline desensitizer material selected from alkali and alkaline earth metal carbonates, bicarbonates, hydroxides and hydrous oxides, and other salts such as the corresponding ammonium salts to prevent detonation when said mixture is burned in a smoldering fire.

The compositions of the invention can be made by simple blending techniques well known to the art for handling explosive materials. For example, the nitrate can be added to a mixture of excipient containing the desensitizer, the desensitizer can be added to a blend of nitrate plus excipient or alternatively all three may be combined at once. The choice of blending method may require different concentrations of desensitizer to gain equivalent results. In a preferred procedure the desensitizer is added to a blend of the nitrate in excipient so that the nitrate particle is coated with the desensitizer solid material.

The organic nitrate/excipient blend can be made by first dissolving the nitrate in a highly volatile solvent such as alcohol, acetone or the like and thereafter adding the nitrate solution to a commercial blender containing either the inert excipient or a blend of the excipient with the desensitizer. One suitable procedure involves adding an acetone solution of nitrate such as nitroglycerin to an evacuated rotating v-shaped blender such as that made by the Patterson-Kelly Corporation. The solvent containing nitrate distributes over the surface of the solid powdered excipient after which the solvent evaporates leaving a fully-blended nitrate-coated solid. A minor amount of the powdered desensitizer may be coated over the nitrate-containing particle in the same blending device. It is further contemplated that the ingredients can be blended in an aqueous paste solution after which the composition may be extruded and dried by conventional techniques. Many other commercially-available blending devices normally acceptable for use with explosives may be employed for making the mixture.

The drug mixtures usually will contain from 5—50% by weight of the organic nitrate dispersed in an inert non-alkaline solid excipient and a minor amount of an alkaline solid material as a desensitizer. Excipient blends of nitroglycerine can be stabilized against detonation when they contain at least about 0.05 parts of alkaline desensitizer per part of organic nitrate. In most instances larger amounts of desensitizer in the blend offer little improvement up to 0.2 parts per part organic nitrate and higher up to equal amounts.

Desensitizing alkaline solid material may be selected from the carbonates, bicarbonates, hydroxides and hydrous oxides of alkali and alkaline earth metals selected from lithium, sodium, potassium, calcium, magnesium and barium. Ammonium carbonate is effective at higher concentration; however, a darkened product results after standing. When lactose is used as an excipient, magnesium hydroxide and carbonate are preferred since they have less tendency to darken the blend. Preferred alkaline materials may be selected from the group consisting of magnesium carbonate, magnesium hydroxide, sodium bicarbonate, sodium carbonate, sodium hydroxide, calcium carbonate and ammonium carbonate.

It is contemplated that any inert non-alkaline organic or inorganic excipient can be employed.

2

Commonly used are such materials as lactose, starch, flour, mannitol, sorbitol, sugar and polyvinylpyrrolidone. Inorganic materials may be selected from clays such as bentonite and attapulgite, silica and the like.

In addition to these essential ingredients coloring agents, flavors or additional medications may be employed.

The following examples are presented to demonstrate the effectiveness of various alkaline solid materials in stabilizing nitroglycerin/lactose blends. However, the equivalent results are expected to be obtained with compositions containing isosorbide dinitrate, isosorbide 5-mononitrate, isosorbide 2-mononitrate, mannitrol hexanitrate and PETN in concentrations ranging from 5 to 50% by weight. In the following examples all proportions are expressed in parts by weight unless otherwise stated.

Preparation A

A solution of 10 parts of nitroglycerin in 90 parts of acetone held in a polyethylene tank was added to 90 parts of hydrous lactose USP held in a commercially-available rotating ribbon type blender turning at low speed. When all the acetone solution had been added, usually over a period of about 5 hours, and thoroughly mixed in the ribbon blender the material was transferred to paper-covered trays held on a drying cart and placed in a drying room for at least 16 hours at about 50°C. The material was then passed through a #10 US Sieve Series screen and stored in polyethylene-lined fiber drums.

Examples

Varying amounts of Preparation A were placed in a ribbon blender and combined with a pulverized alkaline solid desensitizer to form evenly-distributed blends containing 0.05, 0.1 and 0.2 parts of desensitizer per part of nitroglycerine wherein the ratio of lactose to nitroglycerine was 9:1.

Burn tests were conducted for each of the desensitized blends by placing 907.2 g. of nitrate mixture in a 3-mil thick polyethylene bag measuring 15.25 cm. in diameter and 39.48 cm. in length. The open end was tied off to form a chub measuring 15.24 cm. in diameter by 25.4 cm. in length. These samples were then placed in a 3 AF cap carton and taped. 5 samples of each blend including the control were prepared in this way for burn testing.

Each sample was burned at an outdoor test ground as follows:

1 multiwall paperbag (58.42 cm. × 88.9 cm.) having 3 paper layers and 1 polyethylene layer was placed at the bottom of a hole dug in the ground measuring 76.2 cm. × 76.2 cm. square and 15.24 xm. deep. In the center of the bag over a 40.64 cm. × 40.64 cm. area were placed 5 layers of pine-wood furring strips measuring 1.9 cm. × 6.67 cm. × 40.64 cm. 4 strips were placed 10.16 cm. apart parallel to one another on the bag as a first layer. The second layer was placed on top of the furring strips at a 90° angle thereto spaced 10.16 cm. apart. 3 more layers were piled in alternating directions to form the 5 layers of furring strips. 1.89 litres of No. 2 fuel oil were then poured over the wood strips. The 907.2 g. sample was then placed on top of the pile of wood. The fire was ignited and watched from behind a barricade. The wind velocity was no greater than 16 km. per hour. After about 37 to 42 minutes the blend containing no desensitizer had detonated. Test results for the various desensitizer materials tried appear in Table 1.

While concentrations of desensitizer ranging to as low as 0.05 parts per part of nitrate were found to be effective, it is contemplated that even lower quantities may be employed if the alkaline solid is added wet to the ribbon mixer after the nitroglycerine/solvent combination is blended with the lactose, since the desensitizer can be more evenly coated over the lactose/nitrate particle.

While other nitrates such as isosorbide dinitrate, isosorbide 5-mononitrate, isosorbide-2-mononitrate, mannitol hexanitrate and PETN are somewhat less sensitive than nitroglycerine, it is expected that the incorporation of the desensitizer materials will be effective in reducing the detonation hazard if similar detonating conditions arise in blends containing these substances.

TABLE I

| Example No. | Desensitizer | Concentration (parts by wt. per part of nitrate) | Detonation Test Results | |
|---|---|---|---|---|
| | | | No. of Samples | No. of Detonations |
| (Control) | None | 0 | 5 | 5 (37—42 min.) |
| 1 | NaHCO$_3$ | 0.2 | 5 | 0 |
| 2 | NaHCO$_3$ | 0.05 | 5 | 0 |
| 3 | Na$_2$CO$_3$ | 0.2 | 5 | 0 |
| 4 | MgCO$_3$. | 0.2 | 5 | 0 |
| 5 | MgCO$_3$ | 0.05 | 4 | 0 |
| 6 | Mg(OH)$_2$ | 0.2 | 5 | 0 |
| 7 | Mg(OH)$_2$ | 0.05 | 4 | 0 |
| 8 | CaCO$_3$ | 0.2 | 5 | 0 |
| 9 | CaCO$_3$ | 0.05 | 4 | 0 |
| 10 | (NH$_4$)$_2$CO$_3$ | 0.2 | 5 | 0 |

**Claims**

1. An organic nitrate drug mixture comprising
(a) 5—50% by weight of an organic nitrate selected from nitroglycerine, isosorbide dinitrate, isosorbide 5-mononitrate, isosorbide 2-mononitrate, mannitol hexanitrate and pentaerythritol tetranitrate, and
(b) a non-alkaline excipient; characterised in that it further contains
(c) an effective amount of a solid alkaline desensitizer material selected from alkali metal and alkaline earth metal carbonates, bicarbonates, hydroxides and hydrous oxides and the corresponding ammonium salts to prevent detonation when said mixture is burned in a smoldering fire.

2. A composition of claim 1 wherein said solid alkaline material is selected from magnesium carbonate, magnesium hydroxide, sodium bicarbonate, sodium carbonate, sodium hydroxide, calcium carbonate and ammonium carbonate.

3. A composition of Claim 1 wherein said solid alkaline material is present in the mixture in quantities ranging from 0.05—0.2 parts by weight per part by weight of organic nitrate.

4. A composition of Claim 1 wherein said excipient is selected from lactose, starch, flour, mannitol, sorbitol, sugar, polyvinylpyrrolidone, bentonite, attapulgite and silica.

5. A composition of Claim 1 comprising a blend containing 1 part nitroglycerine, 9 parts lactose and, based on one part by weight of nitroglycerin, 0.05—0.2 parts of an alkaline material selected from the group consisting of magnesium carbonate, magnesium hydroxide, sodium bicarbonate, sodium carbonate, sodium hydroxide, calcium carbonate and ammonium carbonate.

**Patentansprüche**

1. Arzneimischung mit einem Gehalt an einem organischen Nitrat, welche folgendes enthält:
(a) 5 bis 50 Gew.-% eines organischen Nitrats, das ausgewählt ist aus Nitroglycerin, Isosorbid-dinitrat, Isosorbid-5-mononitrat, Isosorbid-2-mononitrat, Mannit-hexanitrat und Pentaerythrit-tetranitrat, und
(b) ein nichtalkalisches Exzipiens,
dadurch gekennzeichnet, daß sie weiterhin folgendes enthält:
(c) eine wirksame Menge eines festen alkalischen Desensibilisierungsmaterials, das ausgewählt ist aus Alkalimetall- und Erdalkalimetallcarbonaten, -bicarbonaten, -hydroxiden und wasserhaltigen -oxiden und den entsprechenden Ammoniumsalzen, um eine Detonation zu verhindern, wenn das Gemisch bei einem Schwelbrand angebrannt wird.

2. Zusammensetzung nach Anspruch 1, bei welcher das feste alkalische Material ausgewählt ist aus Magnesiumcarbonat, Magnesiumhydroxid, Natriumbicarbonat, Natriumcarbonat, Natriumhydroxid, Calciumcarbonat und Ammoniumcarbonat.

4

3. Zusammensetzung nach Anspruch 1, bei welcher das feste alkalische Material im Gemisch in Mengen im Bereich von 0,05 bis 0,2 Gew.-Teilen je Gewichtsteil organisches Nitrat vorliegt.

4. Zusammensetzung nach Anspruch 1, bei welcher das Exzipiens ausgewählt ist aus Lactose, Stärke, Mehl, Mannit, Sorbit, Zucker, Polyvinylpyrrolidon, Bentonit, Attapulgit und Siliciumdioxid.

5. Zusammensetzung nach Anspruch 1, welche ein Gemisch darstellt, das 1 Teil Nitroglycerin, 9 Teile Lactose und je Gewichtsteil Nitroglycerin 0,05 bis 0,2 Teile eines alkalischen Materials, das ausgewählt ist aus Magnesiumcarbonat, Magnesiumhydroxid, Natriumbicarbonat, Natriumcarbonat, Natriumhydroxid, Calciumcarbonat und Ammoniumcarbonat, enthält.

**Revendications**

1. Composition pharmaceutique contenant un nitrate organique, renfermant

(a) 5 à 50% en poids d'un nitrate organique choisi entre la nitroglycérine, le dinitrate d'isosorbide, le 5-mononitrate d'isosorbide, le 2-mononitrate d'isosorbide, l'hexanitrate de mannitol et le tétranitrate de pentaérythritol, et

(b) un excipient non alcalin, caractérisée en ce qu'elle contient en outre

(c) une quantité efficace d'une matière alcaline solide servant de désensibilisant choisie entre des carbonates, bicarbonates, hydroxydes et oxydes hydratés de métaux alcalins et de métaux alcalino-terreux, et les sels d'ammonium correspondants, pour empêcher la détonation lorsque ladite composition est soumise à une combustion dans un feu couvant.

2. Composition suivant la revendication 1, dans laquelle la matière alcaline solide est choisie entre le carbonate de magnésium, l'hydroxyde de magnésium, le bicarbonate de sodium, le carbonate de sodium, l'hydroxyde de sodium, le carbonate de calcium et le carbonate d'ammonium.

3. Composition suivant la revendication 1, dans laquelle la matière alcaline solide est présente dans le mélange en des quantités de 0,05 à 0,2 partie en poids par partie en poids de nitrate organique.

4. Composition suivant la revendication 1, dans laquelle l'excipient est choisi entre le lactose, l'amidon, la farine, le mannitol, le sorbitol, le sucre, la polyvinylpyrrolidone, la bentonite, l'attapulgite et la silice.

5. Composition suivant la revendication 1, comprenant un mélange contenant une partie de nitroglycérine, 9 parties de lactose et, sur la base d'une partie en poids de nitroglycérine, 0,05 à 0,2 partie d'un matière alcaline choisie dans le groupe comprenant le carbonate de magnésium, l'hydroxyde de magnésium, le bicarbonate de sodium, le carbonate de sodium, l'hydroxyde de sodium, le carbonate de calcium et le carbonate d'ammonium.